(19) Europäisches Patentamt / European Patent Office / Office européen des brevets

(11) **EP 2 272 581 A1**

(12) **DEMANDE DE BREVET EUROPEEN**

(43) Date de publication:
**12.01.2011 Bulletin 2011/02**

(21) Numéro de dépôt: **10290262.4**

(22) Date de dépôt: **18.05.2010**

(51) Int Cl.:
*B01D 53/02* (2006.01)    *B01D 53/047* (2006.01)
*C01B 3/56* (2006.01)    *C01B 31/00* (2006.01)
*C10L 3/10* (2006.01)    *B01J 20/20* (2006.01)

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **22.06.2009 FR 0903009**

(71) Demandeurs:
• **IFP Energies Nouvelles**
  **92852 Rueil-Malmaison Cedex (FR)**
• **CNRS**
  **75794 Paris Cedex 16 (FR)**

(72) Inventeurs:
• **Pirngruber, Gerhard**
  **69390 Charley (FR)**
• **Jolimaitre, Elsa**
  **69001 Lyon (FR)**
• **Parmentier, Julien**
  **68910 Labaroche (FR)**
• **Ducrot-Boisgontier, Claire**
  **67600 Selestat (FR)**
• **Patarin, Joël**
  **68720 Flaxlanden (FR)**

(54) **Procédé de séparation de CO2 par adsorption modulée en pression sur un solide carbone poreux préparé par nanomoulage**

(57)    On décrit un procédé de séparation de dioxyde de carbone présent dans un mélange gazeux par adsorption modulée en pression (PSA) comprenant au moins les étapes suivantes :

a) la mise en contact dudit mélange à purifier avec au moins un adsorbant formé d'un matériau carboné poreux comprenant au moins 70% poids de carbone, ledit matériau présentant une surface spécifique supérieure à 1500 m²/g, un volume microporeux compris entre 0,5 et 2 cm³/g d'adsorbant et entre 0,5 et 0,8 cm³/cm³ d'adsorbant,

b) l'adsorption du dioxyde de carbone sur ledit adsorbant à une pression totale P1,

c) la désorption d'au moins une partie du $CO_2$ adsorbé à ladite étape b) de manière à produire un flux enrichi en $CO_2$ à une pression totale P2 inférieure à P1.

EP 2 272 581 A1

**Description**

Domaine technique de l'invention

La présente invention se rapporte au domaine de la séparation du dioxyde de carbone présent dans un mélange gazeux à purifier, tel qu'un gaz de synthèse ou le gaz naturel. De manière plus précise, la séparation du dioxyde de carbone est effectuée, par adsorption, par la mise en oeuvre d'un procédé PSA (Pressure Swing Adsorption ou adsorption modulée en pression) avec utilisation d'au moins un matériau carboné microporeux préparé par nanomoulage.

Etat de la technique antérieure

Le procédé d'adsorption modulée en pression ou procédé PSA est un procédé couramment employé dans le domaine de la séparation et de la purification des gaz.

Le principe du procédé PSA est bien connu de l'homme du métier. Il s'agit d'un procédé cyclique, alternant entre des étapes d'adsorption, d'égalisation de pression, de désorption, de purge. En règle générale, le flux gazeux à purifier traverse à la pression haute du cycle, encore appelée « pression d'adsorption » des couches ou lits de matériaux adsorbants qui retiennent préférentiellement, par adsorption, la ou les impureté(s) présentes dans le mélange gazeux à purifier. L'étape d'adsorption conduit à la production d'un gaz purifié, essentiellement constitué d'un composé que l'on cherche à obtenir à l'état pur et qui ne présente que peu d'affinité avec le(s) lit(s) d'adsorbants. Cette (ces) impureté(s) adsorbée(s) sur les adsorbants lors de la mise en oeuvre de l'étape d'adsorption du procédé PSA est (sont) ensuite extraite(s) desdits matériaux adsorbants en baissant la pression jusqu'à une pression dite « de désorption » lors de la mise en oeuvre de l'étape de désorption du procédé PSA. La désorption est d'abord réalisée par l'abaissement de la pression et ensuite par balayage du ou des lit(s) d'adsorbants par un gaz d'élution de sorte que la (les) impureté(s) adsorbées soi(en)t désorbée(s) à la pression de désorption. Ladite étape de désorption conduit à la production d'un flux de gaz résiduaire contenant la (les) impureté(s) désorbée(s). Le principe même du procédé PSA est d'enchaîner cycliquement ces phases d'adsorption à haute pression et de désorption à basse pression, avec éventuellement des étapes supplémentaires d'égalisation de pression et de purge. Le mode de fonctionnement du procédé PSA est rappelé dans la demande de brevet FR-A-2.910.457. Le gaz d'élution est préférentiellement choisi parmi les composés de la charge à purifier.

Les procédés de purification de gaz par PSA sont couramment utilisés dans l'industrie. Un exemple très important est l'emploi du procédé PSA pour la production d'hydrogène de haute pureté (appelé PSA hydrogène) à partir du gaz de synthèse formé d'un mélange comprenant de l'hydrogène, du dioxyde de carbone ($CO_2$) du monoxyde de carbone (CO), du méthane ($CH_4$) et de l'eau ($H_2O$). Un procédé PSA classique pour la purification de l'hydrogène présent dans un gaz de synthèse contient plusieurs lits d'adsorbants. En règle générale, on utilise tout d'abord un lit de charbon actif pour adsorber le dioxyde de carbone et ensuite un lit de zéolithe 5A ou NaX pour adsorber le méthane, le monoxyde de carbone et les autres impuretés éventuellement présentes dans la charge constitué par le gaz de synthèse. Lorsque de l'eau est présente dans la charge, le procédé PSA peut également contenir un lit préliminaire de gel de silice de manière à prétraiter ladite charge et la débarrasser de l'eau. La purification de l'hydrogène en mélange dans un gaz de synthèse par la mise en oeuvre d'un procédé PSA conduit à la production d'un flux enrichi en hydrogène (pureté généralement d'au moins 99,5 % voire supérieure à 99,99%) au cours de l'étape d'adsorption et d'un flux résiduaire enrichi en $CO_2$, CO et $CH_4$ notamment au cours de l'étape de désorption. Pour les PSA hydrogène, le gaz d'élution utilisé industriellement est une partie de l'hydrogène purifié produit lors de l'étape d'adsorption. Mais il est également possible d'utiliser une partie du gaz de purge, tel que décrit dans de nombreux articles (Diagne, D., Goto, M., Hirosi, T., 1995a. "Experimental study of simultaneous removal and concentration of CO2 by an improved pressure swing adsorption process". Energy Conversion and Management 36, 431-434 ; Ebner, A.D., Ritter, J.A., 2004. "Equilibrium theory analysis of dual reflux PSA for separation of a binary mixture". A.I.Ch.E. Journal 50 (10), 2418-2429).

Contrairement aux procédés de purification d'$H_2$ par absorption avec un solvant ou par une séparation membranaire, le procédé PSA permet d'obtenir un flux d'$H_2$ de très grande pureté (> 99.999%). En revanche, l'inconvénient du procédé PSA réside dans son faible rendement en $H_2$ (rapport entre le débit d'hydrogène produit et le débit d'hydrogène contenu dans la charge à purifier) qui est habituellement compris entre 80 et 90%. Les besoins croissants en hydrogène dans les raffineries, les complexes pétrochimiques et les procédés du secteur énergétique (procédés de production d'électricité tels que l'IGCC (cycle combiné à gazéification intégrée), par exemple) imposent une constante recherche en vue d'améliorer la performance des procédés de purification de l'hydrogène par PSA, en particulier leur rendement et leur productivité (c'est-à-dire le débit d'hydrogène produit rapporté au volume ou à la masse d'adsorbant nécessaire à la séparation). Une telle amélioration est possible en travaillant sur le cycle du procédé PSA (à savoir sur l'enchaînement des étapes d'adsorption, de désorption et d'égalisation de pression entre deux colonnes contenant les lits d'adsorbants) et/ou en travaillant sur les propriétés d'adsorption des adsorbants. Le $CO_2$ étant une des impuretés principales du gaz de synthèse duquel on cherche à produire un flux enrichi en hydrogène, une des pistes exploitées en vue d'augmenter la productivité du procédé PSA réside dans la recherche d'une meilleure élimination de cette impureté.

Un autre exemple d'utilisation prépondérante du procédé PSA concerne l'extraction du $CO_2$ présent dans le gaz naturel. En effet, le gaz naturel, en sortie de puits contient en général du $CO_2$, en mélange avec du méthane, des hydrocarbures légers, de l'hydrogène sulfuré ($H_2S$), de l'azote ($N_2$), de l'eau, etc. Or, il est important d'extraire au maximum le $CO_2$ afin de minimiser les débits de gaz à transporter, d'augmenter le pouvoir calorifique du gaz, de permettre la liquéfaction du gaz, d'utiliser le $CO_2$ pour le réinjecter dans les puits de pétrole en vue d'augmenter le taux de récupération du pétrole brut. Les puretés à atteindre sont dépendantes des applications visées. Dans tous les cas, il s'agit de volumes de gaz à traiter très importants, et une amélioration même minime des performances du procédé PSA peut permettre un gain significatif en coûts d'investissement et de fonctionnement. Pour une telle application, le procédé PSA conduit, au cours de la phase d'adsorption, à la production d'un flux enrichi en méthane et au cours de la phase de désorption à un flux résiduaire enrichi en $CO_2$, et éventuellement d'autres impuretés présentes dans le gaz naturel.

Le procédé PSA est également employé avec succès pour extraire le dioxyde de carbone présent dans le biogaz. Le biogaz comprend essentiellement du méthane (au moins 40% du volume) provenant de la fermentation de divers déchets mais contient aussi une part importante de $CO_2$ qu'il convient de séparer du méthane. Dans une telle application, le procédé PSA conduit, au cours de la phase d'adsorption, à la production d'un flux enrichi en méthane et au cours de la phase de désorption à un flux résiduaire enrichi en $CO_2$ et éventuellement d'autres impuretés présentes dans le biogaz, par exemple l'azote.

Un des moyens pour améliorer les performances d'un procédé PSA utilisé pour la séparation de $CO_2$ présent dans un mélange gazeux réside dans l'amélioration des propriétés du solide adsorbant réalisant l'adsorption du $CO_2$. En améliorant les performances d'un adsorbant envers la ou les espèce(s) à adsorber, à savoir envers le $CO_2$ dans le cadre de l'invention, il en découle directement une amélioration des performances du procédé PSA.

Les performances d'un adsorbant sont fonction de deux paramètres principaux : la capacité dynamique d'adsorption et la sélectivité d'adsorption. Dans le cadre de la présente invention, ces paramètres sont examinés eu égard au $CO_2$, à savoir la capacité dynamique d'adsorption en $CO_2$ et la sélectivité d'adsorption envers le $CO_2$.

1. La capacité dynamique d'adsorption en $CO_2$ est définie comme étant la différence entre la quantité de $CO_2$ adsorbée dans les conditions de l'étape d'adsorption et la quantité de $CO_2$ restant adsorbée après l'étape de désorption. La capacité dynamique d'adsorption est une fonction des isothermes d'adsorption des différents constituants de la charge. En négligeant les effets cinétiques, la capacité dynamique d'adsorption d'un constituant donné, par exemple le $CO_2$ dans le cadre de la présente invention, peut être estimée par le calcul de l'écart entre la quantité adsorbée dudit constituant à la pression d'adsorption et la quantité dudit constituant restant adsorbée à la pression de la désorption.

Dans la littérature, les capacités d'adsorption sont souvent exprimées en mol/kg d'adsorbant. En revanche, la grandeur permettant de dimensionner la taille d'une colonne d'adsorption dans un procédé PSA n'est pas la capacité d'adsorption exprimée par masse d'adsorbant, mais la capacité d'adsorption par volume d'adsorbant. La conversion de la capacité d'adsorption exprimée par masse d'adsorbant en capacité d'adsorption exprimée par volume d'adsorbant est effectuée à partir de la densité de la particule d'adsorbant. La densité de la particule peut être mesurée par porosimétrie au mercure (à basse pression) ou être calculée par la formule :

$$\rho_{particule} = \frac{1}{V_{poreux} + 1/\rho_{structurale}},$$

$V_{poreux}$ étant le volume poreux de l'adsorbant et $\rho_{structurale}$ la densité structurale obtenue par pycnométrie.

2. La sélectivité d'adsorption envers le $CO_2$ correspond au rapport entre la quantité de $CO_2$ adsorbée et celle des autres impuretés adsorbées, dans les conditions de fonctionnement du procédé PSA lors de l'étape d'adsorption à haute pression. L'adsorbant optimal est celui qui est le plus sélectif vis à vis de l'impureté que l'on cherche à extraire de la charge.

Pour améliorer l'adsorption sélective du $CO_2$, il s'agit donc d'augmenter la capacité dynamique d'adsorption en $CO_2$ (exprimée en moles adsorbées par unité de volume d'adsorbant) présenté par l'adsorbant et/ou la sélectivité dudit adsorbant vis à vis du $CO_2$. Les performances d'un procédé PSA sont caractérisées principalement par les puretés du(es) produit(s) obtenu(s) lors de la phase d'adsorption, par exemple par la pureté en hydrogène pour un procédé de séparation d'hydrogène présent dans un gaz de synthèse, le rendement du(des) produit(s) obtenu(s) lors de la phase d'adsorption (c'est à dire le débit de sortie du(es)dit(s) produit(s) rapportée aux débits du(es)dit(s) produit(s) contenus dans la charge) et la productivité du procédé PSA correspondant au débit de produit(s) formé(s) lors de la phase d'adsorption rapporté à la masse (ou au volume) d'adsorbant contenue dans le procédé. Ces trois caractéristiques permettant d'évaluer les performances d'un procédé PSA sont liées. La capacité dynamique, qui permet de calculer la quantité de chaque impureté adsorbée au cours d'un cycle d'un procédé PSA, permet d'évaluer la quantité d'adsorbant nécessaire pour extraire la quantité désirée d'impureté, et donc d'atteindre la pureté souhaitée en sortie de procédé. C'est pour cette raison que la capacité dynamique est le principal paramètre dimensionnant pour un procédé PSA. La capacité dynamique impacte ainsi l'ensemble des performances du procédé PSA. En conséquence, une augmentation de la capacité dynamique peut permettre d'augmenter la pureté du(es) produit(s) obtenu(s) lors de la phase d'adsorption, par exemple de l'hydrogène, le rendement du(es) produit(s) obtenu(s) lors de la phase d'adsorption, par exemple le rendement en hydrogène, ou la productivité du procédé, selon le choix de l'opérateur. Dans tous les cas, une augmentation de la capacité dynamique d'adsorption de l'espèce qu'on cherche à adsorber préférentiellement permet d'améliorer les performances du procédé PSA et est donc fortement recherchée.

Les adsorbants classiquement utilisés pour capter le $CO_2$ à forte/moyenne pression (3 - 20 bar de pression partielle de $CO_2$ sont des charbons actifs (voir, par exemple, US 4.171.206). Les propriétés texturales (volume poreux, surface, taille des pores) des charbons actifs peuvent varier fortement en fonction la méthode de préparation (Handbook of Porous Solids, Wiley-VCH, Vol. 3, 2002, p. 1828). En règle générale, la capacité d'adsorption à forte/moyenne pression - exprimée en moles adsorbées par unité de masse d'adsorbant - augmente quand la surface spécifique et le volume poreux augmentent (Frost et coll., J. Phys. Chem. 110, 2006, 9565). Des traitements spéciaux ont été développés pour augmenter le volume poreux et la surface spécifique des charbons actifs (T. Ottowa, R. Tanibata, M. Itoh, Gas Separation Purification 1993, 7, 241). On peut ainsi atteindre un volume poreux de l'ordre de 1,8 $cm^3$/g et une surface spécifique supérieure à 3000 $m^2$/g. En revanche, la densité de ce type de matériau connu sous l'appellation commerciale Maxsorb est très faible (de l'ordre de 0,30 g/$cm^3$). Ainsi, la capacité d'adsorption du $CO_2$ et du $CH_4$ sur le solide Maxsorb (charbon actif conventionnel à forte porosité), exprimée en mol/kg, est nettement plus élevée que celle sur des charbons actifs conventionnels, à partir d'une pression partielle de 2 bar (S. Himeno et al., J. Chem. Eng. Data 50, 2005, 369). En revanche, à cause de la densité très faible de ce matériau, la capacité d'adsorption mesurée en mol/volume d'adsorbant reste comparable aux charbons actifs classiques. Ainsi, les méthodes permettant d'augmenter le volume poreux des adsorbants classiques ne permettent pas d'améliorer les performances de ces adsorbants en terme de capacité d'adsorption.

La porosité de tous les charbons actifs conventionnels est non-organisée. Ce manque d'organisation est inhérent à la préparation des charbons actifs. Ils sont issus de la carbonisation, suivie par des post-traitements, de produits naturels, tels que la houille, le bois ou la noix de coco. Du fait de cette non-organisation, la porosité de ce type de charbon est toujours distribuée et une partie au moins de la porosité est constituée de pores de diamètres peu utiles à la séparation. C'est par exemple le cas des méso- ou macropores (pores de diamètre supérieur à 2nm), dans lesquels les quantités adsorbées sont faibles dans les conditions habituelles de fonctionnement d'un procédé PSA.

Il existe également des charbons "synthétiques", préparés par la carbonisation de précurseurs chimiques. Parmi les charbons synthétiques, les matériaux carbonés préparés par nanomoulage possèdent des propriétés très spécifiques et intéressantes. En particulier, les zéolithes sont des nanomoules particulièrement attractifs, car elles permettent d'obtenir des répliques carbonées microporeuses de porosité contrôlée. Plusieurs zéolithes ont été utilisées pour le nanomoulage, par exemple les zéolithes beta, L, mordenite, ZSM-5, faujasite et EMT. Les meilleures réplications sont obtenues avec des solides ayant un système poreux tri-dimensionnel et une taille de pores supérieure à 0,6 nm (T. Kyotani et al., Carbon 41. 2003, 1451). La méthode de préparation d'une réplique carbonée de la zéolithe faujasite est, par exemple, décrit dans T. Kyotani, Bull. Chem. Soc. Japan, 2006, 79, 1322. Les répliques de la faujasite peuvent atteindre une surface spécifique de 4000 $m^2$/g et un volume microporeux de 1,8 ml/g.

Résumé et intérêt de l'invention

La présente invention a pour objet un procédé de séparation de dioxyde de carbone présent dans un mélange gazeux par adsorption modulée en pression (PSA) comprenant au moins les étapes suivantes :

a) la mise en contact dudit mélange à purifier avec au moins un adsorbant formé d'au moins un matériau carboné poreux comprenant au moins 70% poids de carbone, ledit matériau présentant une surface spécifique supérieure à 1500 $m^2$/g, un volume microporeux compris entre 0,5 et 2 $cm^3$/g d'adsorbant et entre 0,2 et 0,9 $cm^3$/$cm^3$ d'adsorbant,
b) l'adsorption du dioxyde de carbone sur ledit adsorbant à une pression totale P1,
c) la désorption d'au moins une partie du $CO_2$ adsorbé à ladite étape b) de manière à produire un flux enrichi en $CO_2$ à une pression totale P2 inférieure à P1.

Ledit matériau carboné utilisé comme adsorbant dans le procédé de séparation de l'invention est avantageusement préparé selon un mode de préparation utilisant au moins une zéolithe et au moins un composé organique insaturé en tant que réactifs et comprenant au moins une étape de carbonisation et au moins une étape de dissolution de ladite zéolithe. Ledit mode de préparation est un procédé de préparation par nanomoulage qui conduit à l'obtention d'un matériau carboné poreux présentant un volume microporeux compris entre 0,5 et 2,0 cm$^3$/g et compris entre 0,2 et 0,9 cm$^3$/cm$^3$.

Le procédé de séparation selon l'invention est particulièrement avantageux pour la séparation du dioxyde de carbone présent dans un gaz de synthèse ou dans le biogaz en vue de purifier le gaz de synthèse pour produire de l'hydrogène de haute pureté respectivement de purifier le biogaz.

Il a été découvert que, de manière inattendue, ledit adsorbant formé d'un matériau carboné poreux, préparé par nanomoulage et mis en oeuvre dans un procédé PSA pour la séparation de $CO_2$ présent dans un mélange gazeux, présente une capacité dynamique d'adsorption en $CO_2$ sensiblement supérieure à celle des charbons actifs conventionnels commercialisés. Cette amélioration sensible de la capacité dynamique d'adsorption s'observe à la fois pour la quantité de $CO_2$ adsorbée par masse d'adsorbant et pour la quantité de $CO_2$ adsorbée par volume d'adsorbant. Ledit adsorbant formé d'un matériau carboné poreux, préparé par nanomoulage et mis en oeuvre dans le procédé de séparation de $CO_2$ selon l'invention, convient particulièrement pour la mise en oeuvre d'un procédé PSA fonctionnant à pression partielle en $CO_2$ élevée (au moins 3 bar), c'est-à-dire pour des charges riches en $CO_2$ (en général ayant une teneur en $CO_2$ d'au moins 40% molaire) ou à une pression totale élevée (entre 8 et 70 bar). Par ailleurs, ledit adsorbant formé d'un matériau carboné poreux, préparé par nanomoulage et mis en oeuvre dans un procédé PSA pour la séparation de $CO_2$ selon l'invention présente une sélectivité d'adsorption vis-à-vis du $CO_2$ au moins équivalente voire supérieure à celle présentée par les charbons actifs conventionnels commercialisés. Les performances de l'adsorbant formé dudit matériau carboné poreux, préparé par nanomoulage et mis en oeuvre dans un procédé PSA pour la séparation de $CO_2$, étant améliorées eu égard à l'adsorption du $CO_2$ par rapport aux performances d'un adsorbant à base d'un charbon actif conventionnel, il en résulte nécessairement une amélioration des performances du procédé PSA. En particulier, l'augmentation de la capacité dynamique d'adsorption en $CO_2$ présentée par ledit adsorbant formé dudit matériau carboné poreux, préparé par nanomoulage, par rapport à celle présentée par un charbon actif conventionnel permet soit de traiter la même quantité de mélange gazeux à purifier avec un volume d'adsorbant moindre soit de traiter une quantité dudit mélange gazeux plus importante avec un même volume d'adsorbant ; il en découle une amélioration de la productivité du procédé PSA.

Description détaillée de l'invention

La présente invention a pour objet un procédé de séparation de dioxyde de carbone présent dans un mélange gazeux par adsorption modulée en pression (PSA) comprenant au moins les étapes suivantes :

a) la mise en contact dudit mélange à purifier avec au moins un adsorbant formé d'au moins un matériau carboné poreux comprenant au moins 70% poids de carbone, ledit matériau présentant une surface spécifique supérieure à 1500 m$^2$/g, un volume microporeux compris entre 0,5 et 2 cm$^3$/g d'adsorbant et entre 0,2 et 0,9 cm$^3$/cm$^3$ d'adsorbant,
b) l'adsorption du dioxyde de carbone sur ledit adsorbant à une pression totale P1,
c) la désorption d'au moins une partie du $CO_2$ adsorbé à ladite étape b) de manière à produire un flux enrichi en $CO_2$ à une pression totale P2 inférieure à P1.

Ledit matériau carboné poreux utilisé comme adsorbant dans le procédé de séparation de l'invention comprend au moins 70% poids de carbone, de préférence au moins 80% poids de carbone. Il présente une surface spécifique (déterminée par la méthode BET) supérieure à 1500 m$^2$/g, de préférence supérieure à 2000 m$^2$/g et de manière encore plus préférée supérieure à 3000 m$^2$/g. La distribution poreuse dudit matériau carboné est très fine : il présente un volume microporeux compris entre 0,5 et 2,0 cm$^3$/g, de manière préférée compris entre 0,8 et 1,8 cm$^3$/g et de manière encore plus préférée compris entre 0,9 et 1,6 cm$^3$/g. Ce volume microporeux, exprimé en cm$^3$/g, a été déterminé par la méthode de Dubinin-Radushkevich (Dubinin, M. M.; Radushkevich, L. V. Dokl. Acad. Nauk SSSR 1947, 55, 331). Ledit matériau carboné présente un volume microporeux compris entre 0,2 et 0,9 cm$^3$/cm$^3$ (volume poreux par volume d'adsorbant), de manière préférée compris entre 0,5 et 0,8 cm$^3$/cm$^3$. Le volume microporeux, exprimé en cm$^3$/cm$^3$, est obtenu à partir du volume microporeux exprimé en cm$^3$/g et de la densité de l'adsorbant. La densité dudit matériau carboné poreux est préférentiellement comprise entre 0,45 et 0,70 g/cm$^3$ Ledit matériau carboné poreux utilisé comme adsorbant dans le procédé de séparation de l'invention présente avantageusement une porosité majoritairement microporeuse. Les micropores représentent au moins 50 % du volume poreux total, de préférence au moins 65 % du volume poreux total, de manière plus préférée au moins 75% du volume poreux total et de manière encore plus préférée au moins 85% du volume poreux total dudit matériau. De manière très préférée, ledit matériau carboné poreux est avantageusement dépourvu de mésopores. De manière très préférée, il est également dépourvu de macropores. Le volume poreux total dudit matériau est avantageusement compris entre 0,5 cm$^3$/g et 2,5 cm$^3$/g, de préférence entre 0,8 cm$^3$/g et 2,0 cm$^3$/g et de manière encore plus préférée entre 1,0 cm$^3$/g et 1,8 cm$^3$/g. Exprimé en cm$^3$/cm$^3$, ledit volume poreux total est compris entre 0,35 cm$^3$/cm$^3$ et 0,9 cm$^3$/cm$^3$, de préférence entre 0,55 cm$^3$/cm$^3$ et 0,9 cm$^3$/cm$^3$ et de manière encore plus préférée entre 0,65 cm$^3$/cm$^3$ et 0,8 cm$^3$/cm$^3$.

Ledit matériau carboné utilisé comme adsorbant dans le procédé de séparation de l'invention présente avantageusement une porosité organisée à l'échelle des micropores. Plus précisément, il présente des pores organisés à l'échelle microporeuse ayant un diamètre uniforme compris entre 0,5 et 1,5 nm et répartis de façon homogène et régulière dans ledit matériau carboné poreux. Conformément à l'invention, le volume microporeux compris entre 0,5 et 2 cm$^3$/g d'adsorbant, préférentiellement entre 0,8 et 1,8 cm$^3$/g d'adsorbant et entre 0,2 et 0,9 cm$^3$/cm$^3$ d'adsorbant, préférentiellement entre 0,5 et 0,8 cm$^3$/cm$^3$ d'adsorbant, correspond au volume occupé par les pores de diamètre compris entre 0,5 et 1,5 nm. Conformément à l'invention, ledit volume microporeux représente au moins 50 % du volume poreux total, de préférence au moins 65 % du volume poreux total, de manière plus préférée au moins 75% du volume poreux total et de manière encore plus préférée au moins 85% du volume poreux total dudit matériau.

Ledit matériau carboné utilisé comme adsorbant dans le procédé de séparation de l'invention se présente sous forme de poudre ou peut être mis en forme.

Ledit matériau carboné utilisé comme adsorbant dans le procédé de séparation de l'invention est avantageusement préparé selon un mode de préparation utilisant au moins une zéolithe et au moins un composé organique insaturé en tant que réactifs et comprenant au moins une étape de carbonisation et au moins une étape de dissolution de ladite zéolithe. Ledit mode de préparation est un procédé de préparation par nanomoulage : on utilise au moins une zéolithe dont la porosité est dans un premier temps remplie d'au moins une phase organique polymérisée. Ensuite, la zéolithe est dissoute par un traitement acide, créant ainsi une porosité au sein de ladite phase organique laquelle est transformée, par carbonisation, en une matière essentiellement carbonée. Une telle méthode de préparation de matériau carboné poreux a déjà été décrite par T. Kyotani (Bull. Chem. Soc. Japan 2006, 79, 1322). Ladite zéolithe joue ainsi le rôle de nanomoule : le matériau carboné poreux utilisé comme adsorbant dans le procédé de séparation de l'invention est une réplique carbonée négative de ladite zéolithe. Il est avantageux d'utiliser des zéolithes en tant que nanomoule pour la préparation dudit matériau carboné poreux dans la mesure où celles-ci conduisent à l'obtention d'un matériau carboné microporeux. L'utilisation d'une zéolithe ayant une structure cristalline microporeuse parfaitement définie et donc une porosité homogène conduit à l'obtention d'un matériau carboné poreux ayant une porosité bien plus homogène et microporeuse que les charbons actifs classiques. De manière préférée, ledit matériau carboné poreux présente une porosité organisée à l'échelle des micropores. Le degré d'organisation de la porosité du matériau carboné poreux est évalué par diffraction des rayons X, notamment par diffraction des rayons X aux bas angles, cette technique permettant de caractériser la périodicité à l'échelle nanométrique générée par la microporosité organisée dudit matériau carboné. L'analyse des rayons X est réalisée sur poudre avec un diffractomètre opérant en réflexion et équipé d'un monochromateur arrière en utilisant la radiation du cuivre (longueur d'onde de 1,5406A). Les pics habituellement observés sur les diffractogrammes correspondants à une valeur donnée de l'angle 2θ sont associés aux distances inter-réticulaires d$_{(hkl)}$ caractéristiques de la symétrie structurale du matériau ((hkl) étant les indices de Miller du réseau réciproque) par la relation de Bragg : 2 d$_{(hkl)}$ * sin (θ) = n * λ. Cette indexation permet alors la détermination des paramètres (abc) du réseau direct. Pour exemple et de manière très préférée, le diffractogramme de rayons X aux bas angles dudit matériau carboné poreux utilisé en tant qu'adsorbant dans le procédé de séparation selon l'invention présente un pic à une distance d$_{(hkl)}$ proche à celle présente sur le diffractogramme de la zéolithe utilisée en tant que nanomoule. Par "distance d$_{(hkl)}$ proche", on entend au sens de la présente invention une distance comprise entre la distance d$_{(hkl)}$ du pic observé sur le diffractogramme de la zéolithe à ± 10%. L'obtention d'un diffractogramme pour le matériau carboné poreux proche à celui de ladite zéolithe traduit la qualité de mise en oeuvre du procédé de préparation dudit matériau par nonomoulage.

Le procédé de préparation dudit matériau carboné poreux par nanomoulage comprend au moins les étapes :

1) au moins une étape de dépôt chimique en phase vapeur d'au moins un composé organique insaturé sur au moins une zéolithe à une température comprise entre 500 et 800°C,
2) au moins un traitement thermique à une température comprise entre 850 et 1000°C pendant une durée comprise entre 1 et 10 heures,
3) la dissolution de ladite zéolithe dans un excès d'acide fluorhydrique.

Ladite zéolithe utilisée pour la mise en oeuvre de ladite étape 1) est une zéolithe présentant avantageusement un type structural choisi parmi BEA, MOR, MFI, FAU, LTL et EMT et très avantageusement elle présente le type structural FAU ou le type structural EMT. De manière préférée, ladite zéolithe est choisie parmi les zéolites beta, L, mordénite, ZSM-5, faujasite, Y et EMC-2. De manière très préférée, ladite zéolithe est une faujasite ou une zéolithe EMC-2. Ladite zéolithe se présente préférentiellement sous forme sodique (forme Na). Elle est constituée d'au moins un élément trivalent X choisi parmi l'aluminium, le gallium et le mélange de ces deux éléments, de préférence l'aluminium et d'au moins un élément tétravalent Y choisi parmi le silicium, le germanium et le mélange de ces deux éléments, de préférence le silicium. Le rapport molaire Y/X, de préférence le rapport molaire Si/Al, est compris entre 2 et 100, de préférence entre 2 et 10.

Ledit composé organique insaturé utilisé pour la mise en oeuvre de ladite étape 1) est choisi parmi les oléfines, les dioléfines et les alcynes. De manière préférée, ledit composé organique insaturé est choisi parmi le propylène, le butène, le butadiène et l'acétylène. De manière très préférée, ledit composé organique insaturé est le propylène ou l'acétylène.

Ladite étape 1) du procédé de préparation par nanomoulage dudit matériau carboné poreux consiste en la mise en oeuvre d'au moins une étape de dépôt chimique en phase vapeur (CVD) d'au moins dudit composé organique insaturé sur au moins ladite zéolithe. Ladite étape 1) est effectuée à une température comprise entre 500 et 800°C pendant une durée comprise entre 1 et 10 heures. De manière avantageuse, ladite étape 1) est réalisée par la mise en oeuvre de deux étapes de dépôt chimique en phase vapeur dudit composé organique insaturé sur ladite zéolithe, la deuxième étape étant réalisée à une température préférentiellement plus élevée que la première. Le dépôt en phase vapeur dudit composé organique insaturé, de préférence du propylène ou de l'acétylène, dans la porosité de ladite zéolithe est réalisé en présence d'un gaz neutre tel que l'azote ou l'argon. Les quantités dudit composé organique insaturé et de ladite zéolithe introduites pour la mise en oeuvre de ladite étape 1) sont telles que le solide obtenu à l'issue de l'étape 2), explicitée ci-dessous, présente une teneur massique en carbone comprise entre 10 et 25%, de préférence comprise entre 15 et 20%.

Ladite étape 2) du procédé de préparation par nanomoulage dudit matériau carboné poreux consiste en la mise en oeuvre d'au moins un traitement thermique à une température comprise entre 850 et 1000°C pendant une durée comprise entre 1 et 10 heures de manière à carboniser la matière organique introduite dans la porosité de ladite zéolithe au cours de ladite étape 1) et ainsi obtenir la composition carbonée constituant ledit matériau carboné poreux. De manière très préférée, ladite étape 2) est réalisée pendant une durée comprise entre 2 et 5 heures.

Ladite étape 3) du procédé de préparation par nanomoulage dudit matériau carboné poreux consiste en la dissolution de ladite zéolithe dans un excès d'acide fluorhydrique à température ambiante. Par excès d'acide fluorhydrique, on entend une quantité de HF comprise entre 5 et 15 ml par gramme de solide zéolithique. Cette étape conduit à créer la microporosité présente dans ledit matériau carboné poreux. A la suite dudit traitement à l'acide fluorhydrique, le produit est filtré, lavé à l'eau et séché.

La dissolution de ladite zéolithe selon ladite étape 3) est avantageusement suivie d'une étape de lavage à l'acide chlorhydrique afin d'éliminer les phases inorganiques parasites éventuellement produites lors de la dissolution de la zéolithe dans l'acide fluorhydrique. Le traitement à l'acide chlorhydrique est mis en oeuvre en procédant au chauffage à reflux du mélange formé du produit issu de ladite étape 3) et de l'acide chlorhydrique. A la suite dudit traitement à HCl, le produit est filtré, lavé à l'eau et séché. Ledit matériau carboné poreux est obtenu avec les caractéristiques structurales indiquées plus haut dans la présente description.

Selon un mode préféré de réalisation du procédé de préparation par nanomoulage, ladite étape 1) est précédée d'une étape d'imprégnation de ladite zéolithe par au moins un alcool suivie d'une étape de polymérisation dudit alcool dans la porosité de la zéolithe à une température comprise entre 60 et 170°C. Préalablement à l'étape de polymérisation, ladite étape d'imprégnation est préférentiellement immédiatement suivie d'une filtration et d'un lavage du produit mixte zéolithe/alcool afin d'éliminer l'alcool excédentaire déposé à la surface externe de ladite zéolithe. Ladite étape d'imprégnation est avantageusement réalisée à une température comprise entre 15 et 50°C, très avantageusement à température ambiante.

Conformément audit mode préféré de réalisation du procédé de préparation par nanomoulage, l'alcool utilisé est un alcool polymérisable. On utilise avantageusement l'alcool furfurylique ($C_5H_6O_2$). Il est introduit dans une quantité généralement comprise entre 3 et 10 ml par gramme de zéolithe.

Conformément à l'étape a) du procédé de séparation selon l'invention, la mise en contact dudit mélange gazeux à purifier avec au moins ledit adsorbant est effectuée dans au moins une colonne d'adsorption comprenant au moins un lit d'adsorbant, de préférence au moins quatre lits d'adsorbants et de manière encore plus préférée au moins dix lits d'adsorbants, chacun desdits lits comprenant au moins un adsorbant formé dudit matériau carboné poreux préparé par nanomoulage. La colonne est soumise à une étape de pressurisation par ledit gaz jusqu'à atteindre la pression P1 à laquelle l'étape d'adsorption du dioxyde de carbone est mise en oeuvre. Ladite pression totale P1 à laquelle est mise en oeuvre ladite étape d'adsorption b) du procédé de l'invention est préférentiellement comprise entre 5 et 100 bar, préférentiellement comprise entre 5 et 70 bar (1 bar = 0,1 MPa). La pression partielle en $CO_2$ à l'étape d'adsorption, conformément à l'étape b) du procédé selon l'invention, est comprise entre 3 et 50 bar, de préférence entre 3 et 20 bar. Ladite étape b) du procédé de séparation selon l'invention conduit à la production d'un flux gazeux enrichi d'au moins un composé n'ayant pas d'affinité pour ledit adsorbant de sorte qu'il ne s'adsorbe pas ou peu. Ladite étape b) du procédé de séparation selon l'invention est suivie d'une étape de désorption à une pression totale P2 inférieure à P1, ladite pression P2 étant comprise entre 1 et 5 bar. La pression P2 à laquelle ladite étape de désorption est mise en oeuvre est inférieure à la pression partielle de $CO_2$ à laquelle l'étape d'adsorption est mise en oeuvre.

Le procédé de séparation de $CO_2$ selon l'invention est mis en oeuvre à une température de fonctionnement comprise entre 10°C et 250°C, de manière préférée comprise entre 10°C et 100°C et de manière encore plus préférée entre 10°C et 50°C.

Le procédé de séparation selon l'invention trouve très avantageusement un intérêt pour la séparation du dioxyde de carbone présent dans un gaz de synthèse de manière à purifier ledit gaz et produire un flux d'hydrogène de haute pureté. Pour la mise en oeuvre d'un tel procédé de séparation par PSA, ledit gaz de synthèse peut provenir de tout procédé de transformation de charges carbonées en gaz de synthèse, tels que le vaporéformage catalytique, l'oxydation partielle, le réformage autothermique et la gazéification. La composition du mélange gazeux à purifier peut varier en fonction de l'origine du gaz de synthèse. De manière générale, on regroupe sous le terme impuretés les éléments se trouvant en mélange avec l'hydrogène dans la charge, c'est-à-dire principalement le dioxyde de carbone, le monoxyde de carbone et éventuellement le méthane, l'eau, l'azote, l'argon, des hydrocarbures plus lourds en très faible quantité. Toute charge présentant une composition caractérisée par une majeure partie (c'est-à-dire supérieure ou égale à 50% molaire) d'hydrogène, et une mineure partie d'impuretés à base de $CO_2$, CO et éventuellement de méthane, d'eau, d'azote, d'argon et d'hydrocarbures plus lourds, dans des proportions quelconques peut avantageusement constituer une charge du procédé selon la présente invention. Certaines impuretés, en particulier l'eau et l'$H_2S$, sont préférentiellement adsorbées sur un lit d'adsorbant, par exemple une couche de gel de silice, situé en amont d'au moins dudit lit d'adsorbant formé d'au moins dudit matériau carboné poreux de manière à pré-traiter le gaz de synthèse. L'étape b) d'adsorption du procédé selon l'invention conduit à la production d'un flux d'hydrogène de haute pureté tandis que les impuretés, en particulier le dioxyde de carbone, sont adsorbées par le(s) lit(s) d'adsorbants formé d'au moins dudit matériau carboné poreux, préparé par nanomoulage, du(es)quel(s) est(sont) désorbées au moins en partie lesdites impuretés, en particulier le dioxyde de carbone, au cours de la phase de désorption selon ladite étape c) du procédé de l'invention. Il en résulte la production d'un flux résiduaire enrichi en $CO_2$. On rappelle que la différence entre la quantité de $CO_2$ adsorbée dans les conditions de l'étape d'adsorption et la quantité de $CO_2$ restant adsorbée après l'étape de désorption correspond à la capacité dynamique d'adsorption en $CO_2$ de l'adsorbant mis en oeuvre dans le procédé PSA pour l'adsorption et la désorption du $CO_2$. Ledit matériau carboné poreux, préparé par nanomoulage, présente une capacité dynamique d'adsorption en $CO_2$ sensiblement supérieure à celle présentée par les matériaux carbonés conventionnels tels que les charbons actifs.

Le procédé de séparation selon l'invention trouve également très avantageusement un intérêt pour la séparation du dioxyde de carbone présent dans le biogaz comprenant au moins 40 % volume de méthane et au moins 20% volume de $CO_2$. Le biogaz peut également comprendre en plus faible quantité du monoxyde de carbone, de l'azote, de l'hydrogène, de l'oxygène et du sulfure d'hydrogène $H_2S$. Dans une telle application, l'étape b) d'adsorption dudit procédé de séparation selon l'invention conduit à la production d'un flux enrichi en méthane et l'étape c) de désorption conduit à la production d'un flux résiduaire enrichi en $CO_2$ et éventuellement d'autres impuretés.

Le procédé de séparation selon l'invention trouve encore très avantageusement un intérêt pour la séparation du dioxyde de carbone présent dans le gaz naturel. Le gaz naturel comprend essentiellement du méthane (entre 50 et 90% poids environ), du $CO_2$ (entre 1 et 50% poids) et des impuretés telles que les mercaptans, l'$H_2S$, le COS, des hydrocarbures plus lourds et l'eau. Dans une telle application, l'étape b) d'adsorption dudit procédé de séparation selon l'invention conduit à la production d'un flux enrichi en méthane et l'étape c) de désorption conduit à la production d'un flux résiduaire enrichi en $CO_2$ et éventuellement d'autres impuretés telles que celles citées ci-dessus.

Il peut être intéressant de coupler le procédé selon l'invention avec un autre procédé de séparation. Ainsi, on pourra avantageusement augmenter la concentration en $CO_2$ dans le flux résiduaire riche en $CO_2$ par ajout d'un procédé de séparation permettant de séparer sélectivement une partie des molécules contenues dans ce flux tout en maintenant le $CO_2$ en pression. On peut ainsi envisager d'utiliser un procédé membranaire, avec une membrane non-sélective au $CO_2$. Dans ce cas, le $CO_2$ est majoritairement récupéré dans le rétentat, donc à haute pression, alors que les autres composés sont récupérés dans le perméat à plus faible pression.

De même, le couplage avec un autre procédé de séparation peut également permettre d'augmenter la pureté du $CO_2$ contenu dans le flux résiduaire du procédé PSA selon l'invention. Cela peut permettre de réduire les coûts de compression complémentaires, par exemple dans le cas du stockage du $CO_2$. L'augmentation de la pureté du $CO_2$ peut également être intéressante si l'on souhaite utiliser le $CO_2$ produit pour toute application complémentaire. Tout procédé de séparation permettant d'extraire sélectivement une des molécules du flux résiduaire à l'exception du $CO_2$ tout en maintenant le flux riche en $CO_2$ en pression peut être utilisé. On peut citer notamment les procédés membranaires et les procédés d'extraction par solvant.

EXEMPLES

Exemple 1: préparation de matériaux carbonés microporeux par nanomoulage

a) Exemple 1.a. : Synthèse d'un matériau carboné poreux A1 (réplique carbonée microporeuse C-Y2.7-Ac-600/700-4/1-HT)

1 g de zéolithe Na-Y (rapport Si/Al = 2,7) est déposé dans une nacelle en verre de silice et chauffé à 600°C sous flux d'argon (débit de 10 l/h) dans un four. Lorsque la température du four a atteint 600°C, un mélange acétylène/argon (5% d'acétylène en volume - débit total constant = 10 l/h) est envoyé dans le four pendant 4 heures. La température du four est ensuite élevée à 700°C sous argon. Puis un mélange acétylène/argon (5% d'acétylène en volume - débit total constant = 10 l/h) est envoyé dans le four pendant 1 heure. Le four est ensuite placé sous flux d'argon à 900°C et cette température est maintenue constante pendant 4 heures.

Le composite zéolithe/carbone est placé dans l'acide fluorhydrique (40% en masse dans l'eau - 10ml pour 1g de composite) pendant 24 heures à température ambiante. Le solide est filtré, lavé à l'eau et séché à 70°C à l'air pendant une nuit. Cette étape est suivie d'un traitement dans l'acide chlorhydrique (5ml pour 0,1 g de carbone) afin d'éliminer les phases inorganiques qui peuvent se former lors de la dissolution de la zéolithe dans l'acide fluorhydrique. Le produit est mélangé avec l'acide chlorhydrique et chauffé à reflux à 80°C pendant 4 heures puis filtré, lavé à l'eau et séché une nuit à 70°C.

On obtient ainsi un solide carboné microporeux nommé A1. Il est soumis à plusieurs analyses (isotherme d'azote à 77K, pycnométrie à l'hélium, DRX, analyse élémentaire) de manière à déterminer la surface spécifique BET, le volume microporeux exprimé en $cm^3/g$ et $cm^3/cm^3$ ainsi que le volume poreux total exprimé en $cm^3/g$ et $cm^3/cm^3$. Ces caractéristiques structurales de l'adsorbant A1 figurent dans le tableau 1.

L'analyse élémentaire montre que l'adsorbant A1 comprend 81 % poids de carbone.

Le diffractogramme obtenu par l'analyse DRX présente un pic majeur pour 26 = 6,6° soit $d_{hkl}$ = 13,38 Å alors que celui de la zéolithe NaY montre un pic d'intensité maximale pour 2θ = 6,3° soit $d_{hkl}$ = 14,0 Å. La proximité de distance entre le pic observé sur le diffractogramme de A1 et celui de la zéolithe NaY témoigne de la qualité du procédé de préparation du matériau A1 par nanomoulage.

b) Exemple 1.b. : Synthèse d'un matériau carboné poreux A2 (réplique carbonée microporeuse C-Y2.7-AF+Ac-600/700-4/1-HT)

1 g de zéolithe Na-Y (rapport Si/Al = 2,7) est tout d'abord séchée à 150°C puis dégazée sous vide à 300°C pendant 15 h. L'alcool furfurylique (5mL d'alcool pour 1 g de zéolithe, Fluka) est ensuite introduit sous pression réduite et mélangé à la zéolithe pendant 10 minutes à température ambiante. Le mélange est ensuite agité pendant 24 heures sous atmosphère inerte d'azote. Le produit mixte zéolithe/alcool furfurylique est filtré et lavé au mésitylène ($C_3H_3(CH_3)_3$, Fluka) afin d'éliminer l'alcool furfurylique déposé à la surface externe de la zéolithe. Ensuite, l'alcool est polymérisé, sous atmosphère inerte, à 80°C pendant 24 heures et 150°C pendant 8 heures. 1g de composite silice/polyalcool furfurylique est déposé dans une nacelle en verre de silice et chauffé à 600°C sous flux d'argon (débit de 10 l/h) dans un four. Lorsque la température du four a atteint 600°C, un mélange acétylène/argon (5% d'acétylène en volume - débit total constant = 10 l/h) est envoyé dans le four pendant 4 heures. La température du four est ensuite élevée à 700°C sous argon. Puis un mélange acétylène/argon (5% d'acétylène en volume - débit total constant = 10 l/h) est envoyé dans le four pendant 1 heure. Le four est ensuite placé sous flux d'argon à 900°C et cette température est maintenue constante pendant 4 heures.

Le composite zéolithe/carbone est placé dans l'acide fluorhydrique (40% en masse dans l'eau- 10ml pour 1 g de composite) pendant 24 heures à température ambiante. Le solide est filtré, lavé à l'eau et séché à 70°C à l'air pendant une nuit. Cette étape est suivie d'un traitement dans l'acide chlorhydrique (5 ml pour 0,1 g de carbone) afin d'éliminer les phases inorganiques qui peuvent se former lors de la dissolution de la zéolithe dans l'acide fluorhydrique. Le produit est mélangé avec l'acide chlorhydrique et chauffé à reflux à 80°C pendant 4 heures puis filtré, lavé à l'eau et séché une nuit à 70°C.

On obtient ainsi un solide carboné microporeux nommé A2. Il est soumis à plusieurs analyses (isotherme d'azote à 77K, pycnométrie à l'hélium, DRX, analyse élémentaire) de manière à déterminer la surface spécifique BET, le volume microporeux exprimé en $cm^3/g$ et $cm^3/cm^3$ ainsi que le volume poreux total exprimé en $cm^3/g$ et $cm^3/cm^3$. Ces caractéristiques structurales de l'adsorbant A2 figurent dans le tableau 1.

L'analyse élémentaire montre que l'adsorbant A2 comprend 82% poids de carbone.

Le diffractogramme obtenu par l'analyse DRX présente un pic majeur pour 2θ = 6,6° soit $d_{hkl}$ = 13,38 Å alors que celui de la zéolithe NaY montre un pic d'intensité maximale pour 2θ = 6,3° soit $d_{hkl}$ = 14,0 Å. La proximité de distance entre le pic observé sur le diffractogramme de A2 et celui de la zéolithe NaY témoigne de la qualité du procédé de préparation du matériau A2 par nanomoulage.

c) Exemple 1.c : Synthèse d'un matériau carboné A3 (réplique carbonée microporeuse C-Y2.7-AF+Ac-650-5-HT)

1 g de zéolithe Na-Y (rapport Si/Al = 2,7) est tout d'abord séchée à 150°C puis dégazée sous vide à 300°C pendant 15 h. L'alcool furfurylique (5 ml pour 1 g de zéolithe, Fluka) est ensuite introduit sous pression réduite et mélangé à la zéolithe pendant 10 minutes à température ambiante. Le mélange est ensuite agité pendant 24 heures sous atmosphère inerte d'azote. Le produit mixte zéolithe/alcool furfurylique est filtré et lavé au mésitylène ($C_3H_3(CH_3)_3$, Fluka) afin d'éliminer l'alcool furfurylique déposé à la surface externe de la zéolithe. Ensuite, l'alcool est polymérisé, sous atmosphère inerte, à 80°C pendant 24 heures et 150°C pendant 8 heures. 1 g de composite silice/polyalcool furfurylique est déposé dans une nacelle en verre de silice et chauffé à 650°C sous flux d'argon (débit de 10 l/h) dans un four. Lorsque la température du four a atteint 650°C, un mélange acétylène/argon (5% d'acétylène en volume - débit total constant = 10 l/h) est envoyé dans le four pendant 5 heures. Le four est ensuite placé sous flux d'argon à 900°C et cette température est maintenue constante pendant 4 heures.

Le composite zéolithe/carbone est placé dans l'acide fluorhydrique (40% en masse dans l'eau- 10ml pour 1 g de composite) pendant 24 heures à température ambiante. Le solide est filtré, lavé à l'eau et séché à 70°C à l'air pendant une nuit. Cette étape est suivie d'un traitement dans l'acide chlorhydrique (5 ml pour 0,1 g de carbone) afin d'éliminer les phases inorganiques qui peuvent se former lors de la dissolution de la zéolithe dans l'acide fluorhydrique. Le produit est mélangé avec l'acide chlorhydrique et chauffé à reflux à 80°C pendant 4 heures puis filtré, lavé à l'eau et séché une nuit à 70°C.

On obtient ainsi un solide carboné microporeux nommé A3. Il est soumis à plusieurs analyses (isotherme d'azote à 77K, pycnométrie à l'hélium, DRX, analyse élémentaire) de manière à déterminer la surface spécifique BET, le volume microporeux exprimé en $cm^3/g$ et $cm^3/cm^3$ ainsi que le volume poreux total en $cm^3/g$ et $cm^3/cm^3$. Ces caractéristiques structurales de l'adsorbant A3 figurent dans le tableau 1.

L'analyse élémentaire montre que l'adsorbant A3 comprend 81% poids de carbone.

Le diffractogramme obtenu par l'analyse DRX présente un pic majeur pour $2\theta = 6,6°$ soit $d_{hkl} = 13,38$ Å alors que celui de la zéolithe NaY montre un pic d'intensité maximale pour $2\theta = 6,3°$ soit $d_{hkl} = 14,0$ Å. La proximité de distance entre le pic observé sur le diffractogramme de A3 et celui de la zéolithe NaY témoigne de la qualité du procédé de préparation du matériau A3 par nanomoulage.

Tableau 1 : caractéristiques structurales des adsorbants A1, A2 et A3.

| Matériaux carbonés | Surface BET ($m^2/g$) | volume microporeux ($cm^3/g$) | volume microporeux ($cm^3/cm^3$) | volume poreux total ($cm^3/g$) | volume poreux total ($cm^3/cm^3$) |
|---|---|---|---|---|---|
| A1 | 2720 | 1,18 | 0,67 | 1,16 | 0,75 |
| A2 | 3210 | 1,36 | 0,67 | 1,36 | 0,76 |
| A3 | 2260 | 0,95 | 0,57 | 0,85 | 0,71 |

Le tableau 1.1 ci-dessous fait apparaître les caractéristiques structurales des charbons actifs conventionnels testés dans les exemples suivants à titre de comparaison.

Tableau 1.1 : caractéristiques structurales de charbons actifs conventionnels

| Charbons actifs | Surface BET ($m^2/g$) | volume poreux total ($cm^3/g$) | volume poreux total ($cm^3/cm^3$) |
|---|---|---|---|
| Norit R 1 Extra (Norit) | 1450 | 0,47 | 0,20 |
| BPL (Calgon Corp.) | 1150 | 0,43 | 0,21 |
| Maxsorb (Kansai Coke and Chemicals) | 3250 | 1,79 | 0,52 |
| A10 fiber | 1200 | 0,53 | 0,11 |
| Activated carbon A | 1207 | 0,54 | 0,32 |

La société Supelco commercialise des tamis moléculaires carbonés dont le tamis référencé C-1021 qui présente une taille de pores inférieure à 0,56 nm, une surface BET égale à 695 $m^2/g$, un volume microporeux égal à 0,28 $cm^3/g$ et un volume poreux total égal à 0,35 $cm^3/g$. Ce tamis commercial C-1021, bien que présentant une porosité majoritairement microporeuse, présente un volume microporeux, exprimé en $cm^3/g$, faible.

Exemple 2

Cet exemple illustre la capacité dynamique d'adsorption en $CO_2$ de chacun des matériaux carbonés microporeux A1, A2 et A3, préparés dans l'exemple 1, et celle de charbons actifs conventionnels.

La capacité dynamique d'adsorption en $CO_2$ est évaluée expérimentalement par des mesures de "courbes de perçage" : le mélange gazeux constitué de $CO_2$ et d'un gaz inerte (azote) est injecté à 25°C avec un débit donné dans une colonne contenant un lit d'adsorbant A1, respectivement d'adsorbant A2 ou A3. Les concentrations en sortie de chacun des constituants de la charge sont mesurées au cours du temps, jusqu'à ce que l'ensemble des concentrations se stabilisent à leurs valeurs d'entrées (saturation du lit d'adsorbant). Les courbes représentant les concentrations des constituants en fonction du temps sont appelées "courbes de perçage". Le temps moyen de sortie de la courbe de perçage du $CO_2$ permet de calculer sa quantité adsorbée, par la méthode bien connue dite "des moments" (Ruthven, D.M. Principles of adsorption and adsorption processes. John Wiley & Sons ed, 1984). On évalue la capacité dynamique de l'adsorbant A1, respectivement de l'adsorbant A2 et A3 en répétant cette opération à la pression haute (pression d'adsorption) et à la pression basse (pression de désorption) du procédé, et en soustrayant la quantité de $CO_2$ adsorbée à la pression basse à celle adsorbée à la pression haute. Les résultats figurent dans le tableau 2.

On reproduit le test décrit ci-dessus pour la détermination de la capacité dynamique d'adsorption en $CO_2$ de charbons actifs conventionnels. Les résultats figurent dans le tableau 2.

Le tableau 2 suivant présente les valeurs de capacité dynamique d'adsorption en $CO_2$ pour une pression partielle de $CO_2$ en adsorption de 5 bar et une pression partielle de $CO_2$ en désorption de 1 bar.

Tableau 2 : capacités dynamiques d'adsorption en $CO_2$ des matériaux carbonés microporeux A1, A2 et A3 (invention) et de charbons actifs conventionnels entre 5 et 1 bar

| matériaux carbonés | capacité dynamique $CO_2$ (mmol/cm$^3$) |
|---|---|
| A1 | 4,3 |
| A2 | 3,35 |
| A3 | 3,3 |
| Norit R 1 Extra | 1,6 |
| BPL | 1,2 |
| Maxsorb | 1,7 |
| A10 fiber | 0,7 |
| Activated carbon A | 1,8 |

Les résultats figurant dans le tableau 2 démontrent que les matériaux carbonés microporeux A1, A2 et A3 préparés par nanomoulage présentent une capacité dynamique d'adsorption (en mol/cm$^3$) sensiblement supérieure à celle de tous les charbons actifs conventionnels. Comme explicité plus haut dans la présente description, les performances d'un procédé PSA étant directement proportionnelles à la capacité dynamique d'adsorption en $CO_2$, il en résulte que la productivité d'un procédé PSA employé pour la séparation du $CO_2$ présent dans un mélange gazeux sera plus élevée avec les adsorbants A1, A2 et A3 qu'avec les adsorbants à base de charbons actifs conventionnels.

Exemple 3

De même que dans l'exemple 2, cet exemple 3 illustre la capacité dynamique d'adsorption en $CO_2$ de chacun des matériaux carbonés microporeux A1, A2 et A3, préparés dans l'exemple 1, et celle de charbons actifs conventionnels. Dans cet exemple, le procédé PSA a été mis en oeuvre à une pression d'adsorption correspondant à la pression partielle en $CO_2$ égale à 10 bar et une pression de désorption égale à 2 bar. La température de fonctionnement du PSA est égale à 25°C.

La capacité dynamique d'adsorption en CO2 des adsorbants A1, A2 et A3 est déterminée d'une manière analogue à celle décrite dans l'exemple 2, conformément à la méthode bien connue dite "des moments". Les résultats sont reportés dans le tableau 3.

Le tableau 3 présente également la capacité dynamique d'adsorption en $CO_2$ de charbons actifs conventionnels, testés dans les mêmes conditions de fonctionnement du PSA ($P_{CO2\ adsorption}$ = 10 bar, $P_{désorption}$ = 2 bar, T = 25°C). La détermination de la capacité dynamique d'adsorption en $CO_2$ de chacun des charbons actifs conventionnels a été réalisée d'une manière analogue à celle décrite dans l'exemple 2.

Tableau 3 : capacités dynamiques d'adsorption en $CO_2$ des matériaux carbonés microporeux A1, A2 et A3 (invention) et de charbons actifs conventionnels entre 10 et 2 bar

| matériaux carbonés | capacité dynamique $CO_2$ (mmol/cm$^3$) |
|---|---|
| A1 | 5,2 |
| A2 | 5,0 |
| A3 | 6,0 |
| Norit R 1 Extra | 1,4 |
| BPL | 1,2 |
| Maxsorb | 2,0 |
| A10 fiber | 0,6 |
| Activated carbon A | 1,9 |

De même que pour l'exemple 2, les résultats figurant dans le tableau 3 démontrent que les matériaux carbonés microporeux A1, A2 et A3 préparés par nanomoulage présentent une capacité dynamique d'adsorption (en mol/cm$^3$) sensiblement supérieure à celle de tous les charbons actifs conventionnels. De plus, l'augmentation de la pression d'adsorption et de la pression de désorption conduit à une capacité dynamique d'adsorption en $CO_2$ accrue de chacun des adsorbants A1, A2 et A3 alors que les charbons actifs conventionnels ne présentent pas une meilleure capacité dynamique d'adsorption avec l'augmentation de la pression d'adsorption et de la pression de désorption. Cet exemple met en évidence que les solides carbonés microporeux A1, A2 et A3 peuvent être mis en oeuvre dans un procédé PSA fonctionnant à une pression d'adsorption et une pression de désorption élevées.

Exemple 4

Cet exemple illustre la sélectivité d'adsorption vis-à-vis du $CO_2$ de chacun des matériaux carbonés microporeux A1, A2 et A3 et celle de charbons actifs conventionnels, testés dans l'adsorption du $CO_2$ et du $CH_4$. La sélectivité correspond au rapport de la quantité de $CO_2$ adsorbée sur un adsorbant donné sur la quantité de $CH_4$ adsorbée sur ce même adsorbant. Dans cet exemple, la sélectivité a été calculée par le rapport des constantes de Henry du $CO_2$ et du $CH_4$. La constante de Henry du $CO_2$ représente la pente initiale de l'isotherme d'adsorption de $CO_2$ et la constante de Henry du $CH_4$ représente la pente initiale de l'isotherme d'adsorption du $CH_4$.

Le tableau 4 présente la sélectivité de chacun des matériaux carbonés microporeux A1, A2 et A3 ainsi que celle de charbons actifs conventionnels envers le $CO_2$ par rapport au $CH_4$.

Tableau 4 : sélectivité $CO_2/CH_4$ des matériaux carbonés microporeux A1, A2 et A3 (invention) et de charbons actifs conventionnels

| matériaux carbonés | Sélectivité $CO_Z/CH_4$ |
|---|---|
| A1 | 2,9 $\pm$ 0,5 |
| A2 | 3,0 $\pm$ 2 |
| A3 | 3,0 $\pm$ 0,7 |
| Norit R 1 Extra | 1,9 |
| Maxsorb | 1,9 |
| A10 fiber | 2,2 |
| Activated carbon A | 2,2 |

Les résultats figurant dans le tableau 4 démontrent que les matériaux carbonés microporeux A1, A2 et A3 préparés par nanomoulage présentent une sélectivité envers le $CO_2$ équivalente voire supérieure à celle des charbons actifs conventionnels. Or les exemples 2 et 3 ont démontré que lesdits matériaux carbonés microporeux présentent une capacité dynamique d'adsorption en $CO_2$ sensiblement améliorée par rapport à celle des charbons actifs conventionnels. Il en résulte donc une amélioration de la productivité d'un procédé PSA mettant en oeuvre la séparation de $CO_2$ présent dans un mélange gazeux.

Exemple 5 :

Les performances du procédé PSA selon l'invention sont vérifiées dans cet exemple pour la séparation de $CO_2$ présent dans un gaz de synthèse issu du vaporéformage du méthane. La composition du mélange gazeux à purifier est la suivante (% molaire) : 65% $H_2$, 25% $CO_2$, 5% CO et 5% $CH_4$.

Ledit mélange gazeux est purifié en passant au travers d'une colonne contenant un lit d'adsorbant A1, respectivement d'adsorbant A2 ou A3. La pression d'adsorption totale est égale à 24 bar, l'étape de désorption est effectuée à 1 bar et la température de fonctionnement du PSA est égale à 25°C. A l'étape de désorption, on récupère, pour chaque test mis en oeuvre sur chacun des adsorbants A1, A2 et A3, un flux constitué de $92 \pm 1\%$ molaire $CO_2$, $6 \pm 1$ % molaire $CH_4$ et $2 \pm 1$ % molaire CO à 1 bar.

Les quantités de $CO_2$, $CH_4$ et CO co-adsorbées sur chacun des matériaux carbonés microporeux A1, A2 et A3 dans les conditions de l'étape d'adsorption et de l'étape de désorption ont été calculées en utilisant la théorie des solutions idéales (Myers, Prausnitz, AICHE Journal, vol. 11, 1965, 121). On considère que l'hydrogène ne s'adsorbe pas. Les résultats figurent dans le tableau 5.

Le tableau 5 présente également la capacité dynamique d'adsorption en $CO_2$ du charbon actif commercial BPL utilisé, à titre de comparaison, dans la séparation de $CO_2$ présent dans un gaz de synthèse ayant la composition donnée ci-dessus. Le BPL est couramment utilisé dans des procédés PSA (voir, par exemple, US 4,171,206). La capacité dynamique en $CO_2$, égale à 1,1 mmol/cm$^3$, a été calculée à partir des données de la littérature (Wilson, Danner, J. Chem. Eng. Data 1983, 28, 14).

Tableau 5 : capacité dynamique d'adsorption en $CO_2$ en mélange avec $CH_4$, CO et $H_2$ des matériaux carbonés microporeux A1, A2 et A3 (invention) et d'un charbon actif commercial

| matériaux carbonés | Capacité dynamique en $CO_2$ (mmol/cm$^3$) |
|---|---|
| A1 | 3,0 |
| A2 | 2,9 |
| A3 | 2,75 |
| BPL | 1,1 |

Les résultats figurant dans le tableau 5 démontrent que même en mélange avec le méthane, le monoxyde de carbone et l'hydrogène, la capacité dynamique d'adsorption en $CO_2$ de chacun des matériaux carbonés microporeux A1, A2 et A3 est bien meilleure que celle du charbon actif commercial BPL (Wilson, Danner, J. Chem. Eng. Data 1983, 28, 14). Cette différence de capacité dynamique en $CO_2$ se traduira par une amélioration sensible des performances d'un procédé PSA employé pour la séparation du $CO_2$ présent dans le gaz de synthèse.

Exemple 6

Cet exemple illustre la séparation du $CO_2$ présent dans le biogaz par un procédé PSA. La composition volumique du biogaz est la suivante : 54% $CH_4$, 42% $CO_2$ et 4% $N_2$. Des traces d'autres impuretés ($H_2S$, composés organiques volatiles, etc.) peuvent être présentes dans le biogaz mais ont été éliminées de la composition du biogaz en amont de la mise en oeuvre du procédé PSA.

Le biogaz est purifié en passant au travers d'une colonne contenant un lit d'adsorbant A1, respectivement d'adsorbant A2 ou A3. La pression d'adsorption totale est égale à 8 bar, l'étape de désorption est effectuée à 1 bar et la température de fonctionnement du PSA est égale à 25°C. A l'étape de désorption, on récupère, pour chaque test mis en oeuvre sur chacun des adsorbants A1, A2 et A3, un flux constitué de 62-72% vol $CO_2$, 27-37% vol $CH_4$ et <1% vol $N_2$ à 1 bar.

Les quantités de $CO_2$ et $CH_4$ co-adsorbées sur chacun des matériaux carbonés microporeux A1, A2 et A3 dans les conditions de l'étape d'adsorption et de l'étape de désorption ont été calculées en utilisant la théorie des solutions idéales (Myers, Prausnitz, AICHE Journal, vol. 11, 1965, 121). Les résultats figurent dans le tableau 6.

Tableau 6 : capacité dynamique d'adsorption en $CO_2$ en mélange avec $CH_4$ et $N_2$ des matériaux carbonés microporeux A1, A2 et A3

| matériaux carbonés | Capacité dynamique en $CO_2$ (mmol/cm$^3$) |
|---|---|
| A1 | 2,2 |

(suite)

| matériaux carbonés | Capacité dynamique en $CO_2$ (mmol/cm³) |
|---|---|
| A2 | 2,2 |
| A3 | 2,0 |

Les résultats figurant dans le tableau 6 démontrent que chacun desdits matériaux carbonés microporeux A1, A2 et A3 présentent une capacité dynamique d'adsorption en $CO_2$ satisfaisante. Ces résultats mettent en évidence l'intérêt à utiliser lesdits matériaux carbonés microporeux dans un procédé PSA employé pour la mise en oeuvre de la séparation du $CO_2$ présent dans le biogaz.

**Revendications**

1. Procédé de séparation de dioxyde de carbone présent dans un mélange gazeux par adsorption modulée en pression (PSA) comprenant au moins les étapes suivantes :

   a) la mise en contact dudit mélange à purifier avec au moins un adsorbant formé d'au moins un matériau carboné poreux comprenant au moins 70% poids de carbone, ledit matériau présentant une surface spécifique supérieure à 1500 m²/g, un volume microporeux compris entre 0,5 et 2 cm³/g d'adsorbant et entre 0,2 et 0,9 cm³/cm³ d'adsorbant, les micropores représentant au moins 75% du volume poreux total dudit matériau,
   b) l'adsorption du dioxyde de carbone sur ledit adsorbant à une pression totale P1,
   c) la désorption d'au moins une partie du $CO_2$ adsorbé à ladite étape b) de manière à produire un flux enrichi en $CO_2$ à une pression totale P2 inférieure à P1.

2. Procédé de séparation selon la revendication 1 tel que ledit matériau carboné poreux présente un volume microporeux compris entre 0,8 et 1,8 cm³/g.

3. Procédé de séparation selon la revendication 1 ou la revendication 2 tel que ledit matériau carboné poreux présente un volume microporeux compris entre 0,9 et 1,6 cm³/g.

4. Procédé de séparation selon l'une des revendications 1 à 3 tel que ledit matériau carboné poreux présente un volume microporeux compris entre 0,5 et 0,8 cm³/cm³.

5. Procédé de séparation selon l'une des revendications 1 à 4 tel que ledit matériau se présente sous forme de poudre ou est mis en forme.

6. Procédé de séparation selon l'une des revendications 1 à 5 tel que ledit matériau carboné poreux est préparé selon un procédé comprenant au moins les étapes :

   1) au moins une étape de dépôt chimique en phase vapeur d'au moins un composé organique insaturé sur au moins une zéolithe à une température comprise entre 500 et 800°C,
   2) au moins un traitement thermique à une température comprise entre 850 et 1000°C pendant une durée comprise entre 1 et 10 heures,
   3) la dissolution de ladite zéolithe dans un excès d'acide fluorhydrique.

7. Procédé de séparation selon la revendication 6 tel que ladite zéolithe utilisée pour la mise en oeuvre de ladite étape 1) est une zéolithe présentant un type structural choisi parmi BEA, MOR, MFI, FAU, LTL et EMT.

8. Procédé de séparation selon la revendication 6 ou la revendication 7 tel que ledit composé organique insaturé utilisé pour la mise en oeuvre de ladite étape 1) est choisi parmi les oléfines, les dioléfines et les alcynes.

9. Procédé de séparation selon l'une des revendications 6 à 8 tel que ladite étape 1) est précédée d'une étape d'imprégnation de ladite zéolithe par au moins un alcool suivie d'une étape de polymérisation dudit alcool dans la porosité de la zéolithe à une température comprise entre 60 et 170°C.

10. Procédé de séparation selon la revendication 9 tel que ledit alcool est l'alcool furfurylique.

**11.** Procédé de séparation selon l'une des revendications 1 à 10 tel que ladite pression totale P1 mise en oeuvre pour ladite étape d'adsorption b) est comprise entre 5 et 100 bar.

**12.** Procédé de séparation selon l'une des revendications 1 à 11 tel que ladite pression totale P2 est comprise entre 1 et 5 bar.

**13.** Procédé de séparation selon l'une des revendications 1 à 12 tel que ledit mélange gazeux est un gaz de synthèse.

**14.** Procédé de séparation selon l'une des revendications 1 à 12 tel que ledit mélange gazeux est le biogaz comprenant au moins 40% volume de méthane et au moins 20% volume de $CO_2$.

**15.** Procédé de séparation selon l'une des revendications 1 à 12 tel que ledit mélange gazeux est le gaz naturel.

Europäisches
Patentamt

European
Patent Office

Office européen
des brevets

**RAPPORT DE RECHERCHE EUROPEENNE**

Numéro de la demande

EP 10 29 0262

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | DE 26 50 381 A1 (BERGWERKSVERBAND GMBH) 11 mai 1978 (1978-05-11) * revendications 1-3; figure 1 * * page 6, alinéa 1 - alinéa 3 * * page 8, alinéa 2 - page 9, alinéa 3 * ----- | 1-15 | INV. B01D53/02 B01D53/047 C01B3/56 C01B31/00 C10L3/10 B01J20/20 |
| X,D | FR 2 910 457 A (INST FRANCAIS DU PETROLE [FR]) 27 juin 2008 (2008-06-27) * revendications 1,3,10 * * page 6, ligne 10 - page 8, ligne 16 * ----- | 1 | |
| X | EP 1 120 149 A (AIR LIQUIDE [FR]) 1 août 2001 (2001-08-01) * alinéas [0001], [0006], [0031] * * exemple 1 * * revendication 14 * ----- | 1 | |
| A | GUAN C ET AL: "Investigation of H2 storage in a templated carbon derived from zeolite Y and PFA" SEPARATION AND PURIFICATION TECHNOLOGY, ELSEVIER SCIENCE, AMSTERDAM, NL, vol. 66, no. 3, 7 mai 2009 (2009-05-07), pages 565-569, XP026052447 ISSN: 1383-5866 [extrait le 2009-02-04] * figure 2c; tableau 2 * * page 565, colonne 1 - colonne 2 * * page 567, colonne 1 - colonne 2 * ----- | 6-10 | DOMAINES TECHNIQUES RECHERCHES (IPC) B01D C01B C10L B01J |

Le présent rapport a été établi pour toutes les revendications

| Lieu de la recherche | Date d'achèvement de la recherche | Examinateur |
|---|---|---|
| Munich | 29 septembre 2010 | Burkhardt, Thorsten |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 10 29 0262

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-09-2010

| Document brevet cité au rapport de recherche | | Date de publication | Membre(s) de la famille de brevet(s) | | Date de publication |
|---|---|---|---|---|---|
| DE 2650381 | A1 | 11-05-1978 | AUCUN | | |
| FR 2910457 | A | 27-06-2008 | CA | 2671389 A1 | 10-07-2008 |
| | | | CN | 101568369 A | 28-10-2009 |
| | | | EP | 2106284 A2 | 07-10-2009 |
| | | | WO | 2008081102 A2 | 10-07-2008 |
| | | | JP | 2010513201 T | 30-04-2010 |
| | | | KR | 20090091327 A | 27-08-2009 |
| | | | US | 2010089233 A1 | 15-04-2010 |
| EP 1120149 | A | 01-08-2001 | FR | 2804042 A1 | 27-07-2001 |
| | | | US | 2001009125 A1 | 26-07-2001 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2910457 A

- US 4171206 A

**Littérature non-brevet citée dans la description**

- **Diagne, D. ; Goto, M. ; Hirosi, T.** Experimental study of simultaneous removal and concentration of CO2 by an improved pressure swing adsorption process. *Energy Conversion and Management,* 1995, vol. 36, 431-434
- **Ebner, A.D. ; Ritter, J.A.** Equilibrium theory analysis of dual reflux PSA for separation of a binary mixture. *A.I.Ch.E. Journal,* 2004, vol. 50 (10), 2418-2429
- Handbook of Porous Solids. Wiley-VCH, 2002, vol. 3, 1828
- **Frost.** *J. Phys. Chem.,* 2006, vol. 110, 9565
- **T. Ottowa ; R. Tanibata ; M. Itoh.** *Gas Separation Purification,* 1993, vol. 7, 241

- **S. Himeno et al.** *J. Chem. Eng. Data,* 2005, vol. 50, 369
- **T. Kyotani et al.** *Carbon,* 2003, vol. 41, 1451
- **T. Kyotani.** *Bull. Chem. Soc. Japan,* 2006, vol. 79, 1322
- **Dubinin, M. M. ; Radushkevich, L. V.** *Dokl. Acad. Nauk SSSR,* 1947, vol. 55, 331
- **Ruthven, D.M.** Principles of adsorption and adsorption processes. John Wiley & Sons, 1984
- **Myers, Prausnitz.** *AICHE Journal,* 1965, vol. 11, 121
- **Wilson, Danner.** *J. Chem. Eng. Data,* 1983, vol. 28, 14